Europäisches Patentamt

European Patent Office

Office européen des brevets

(11) Publication number: **0 121 215 B1**

# EUROPEAN PATENT SPECIFICATION

(45) Date of publication of patent specification: **06.12.89**

(51) Int. Cl.⁴: **A 61 B 1/12, A 61 B 17/22, A 61 M 25/00**

(21) Application number: **84103360.8**

(22) Date of filing: **27.03.84**

(54) Catheter.

(30) Priority: 31.03.83 JP 56629/83
26.09.83 JP 178730/83
16.12.83 JP 238329/83

(43) Date of publication of application:
**10.10.84 Bulletin 84/41**

(45) Publication of the grant of the patent:
**06.12.89 Bulletin 89/49**

(84) Designated Contracting States:
**DE FR GB IT NL SE**

(56) References cited:
EP-A-0 102 685      DE-A-3 025 785
WO-A-83/03188      DE-A-3 131 652
DE-A-2 813 750      US-A-3 866 599
DE-A-2 834 956      US-A-4 299 226
DE-A-2 847 633

TECHNOLOGICAL DIGESTS, vol. 9, no. 7, July
1964, pages 25,26, USA; "On-and-off and
steerable medical magnets"

(73) Proprietor: SUMITOMO ELECTRIC INDUSTRIES
LIMITED
No. 15, Kitahama 5-chome Higashi-ku
Osaka-shi Osaka 541 (JP)

(73) Proprietor: Inoue, Kiyoshi
Omagi 425,2
Urawa City Saitama (JP)

(73) Proprietor: Kuwaki, Keiichi
Takaido Higashi 4-19-14 Suginami-ku
Tokyo (JP)

(72) Inventor: Inoue, Kiyoshi
Omagi 425-2
Urawa City Saitama (JP)
Inventor: Kuwaki, Keiichi
Takaido Higashi 4-19-14 Suginami-ku
Tokyo (JP)
Inventor: Tsuno, Koichi c/o Osaka Works
Sumitomo
Electr. Ind. Ltd. 1-3, Shimaya 1-chome
Konohana-ku Osaka (JP)

(74) Representative: Glawe, Delfs, Moll & Partner
Patentanwälte
Postfach 26 01 62 Liebherrstrasse 20
D-8000 München 26 (DE)

Courier Press, Leamington Spa, England.

## Description

The present invention relates to a catheter comprising a flexible tube having a fluid passageway for a fluid, said flexible tube being a porous tube made of fluoroethylene resin, and a pressure-resistant rubber covering for covering said flexible tube except for an end portion thereof.

Such a catheter is known e.g. from DE—A—2 813 750.

This catheter has the disadvantage, that in case that a thrombus should occur in the way of the catheter it is hard for the known catheter to pass through this thrombus.

The invention therefore has the object to present a catheter which is possible to dissolve a thrombus in a blood vessel.

According to the invention this object is solved by the feature that a guide wire has its rear end inserted into a front end of said flexible tube and secured thereto so as to protrude therefrom.

The guide wire according to the invention passes first through the thrombus. Thereby it is possible to further advance the catheter until the uncovered end of the chemical delivery tube of the catheter is inserted into the thrombus.

Details of the present invention are hereinafter described with reference to the accompanying drawings in which:

Fig. 1 is a side view of a catheter according to the present invention;

Fig. 2 is an enlarged view of an end portion thereof and

Fig. 3 is a view showing how it is used.

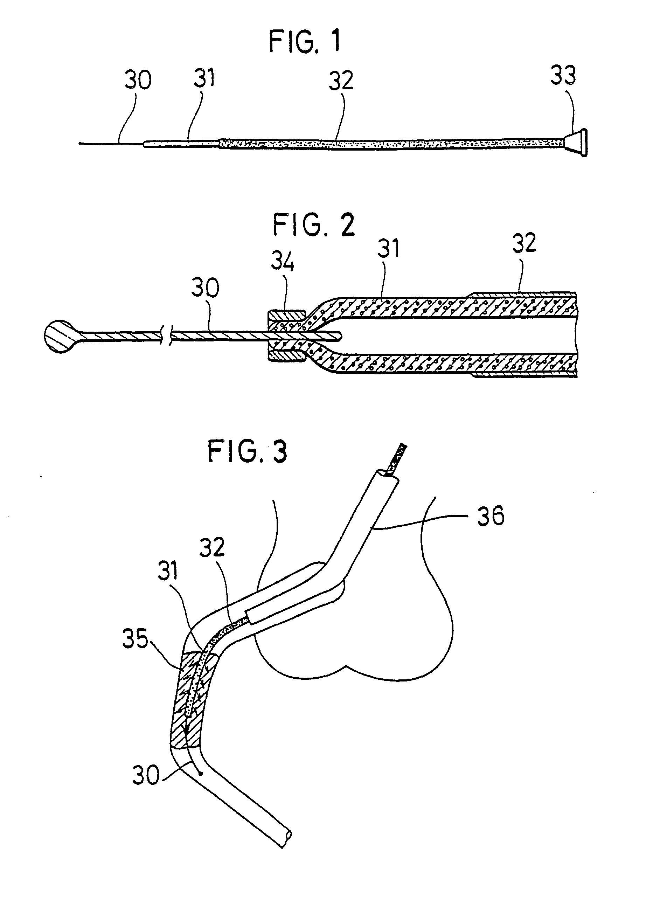

Referring to Figs. 1 to 3, an embodiment of the present invention includes a guide wire 30, a chemical delivery tube 31, a covering 32, and a coupler 33 for coupling a syringe. As shown in Fig. 2 the chemical delivery tube 31 is a porous tube made of a material such as ethylene tetrafluoride resin. To the end of the chemical delivery tube 31, a guide wire 30 is secured by means of a neck ring 34. Except for the end portion extending for about 10 to 20 mm, the chemical delivery tube 31 is covered with the covering 32 made of a material such as fluororubber and silicicrubber. The covering 32 should be capable of resisting the pressure for injecting a thrombus dissolving agent so that the agent will be delivered only from the uncovered end of the chemical delivery tube 31. The covered portion has an outside diameter of about 1 mm.

In order to dissolve a thrombus 35, the catheter in accordance with this embodiment is passed through a cardiac catheter 36 as shown in Fig. 3. Firstly the guide wire 30 passes through the thrombus 35. The catheter in accordance with the invention is further advanced until the uncovered end of the chemical delivery tube 31 is inserted into the thrombus 35. Then the thrombus dissolving agent is injected through the coupler 33 and delivered from the uncovered end of the chemical delivery tube 31. Dissolution occurs over a wide range of the thrombus.

The above-described embodiment has the advantages that since dissolution occurs over a wide range of the thrombus, high efficiency in medical treatment is provided, that the danger of undissolved thrombus getting off the wall of a blood vessel and blocking the blood vessel is minimized, and that the catheter is antithrombotic because the chemical delivery tube is made of a fluorocarbon resin.

## Claims

1. A catheter comprising a flexible tube having a fluid passageway for a fluid, said flexible tube being a porous tube made of fluoroethylene resin, and a pressure-resistant rubber covering for covering said flexible tube except for an end portion thereof, characterized in that a guide wire (30) has it rear end inserted into a front end of said flexible tube and secured thereto so as to protrude therefrom.

2. The catheter as claimed in claim 1, wherein said guide wire (30) has a round end.

## Patentansprüche

1. Katheder bestehend aus einem flexiblen Rohr mit einem Fluiddurchlaß für Fluid, wobei das flexible Rohr ein poröses, aus Fluorethylenharz hergestelltes Rohr ist, und einer druckbeständigen Gummiabdeckung zum Abdecken des flexiblen Rohres mit Ausnahme dessen Endteils, dadurch gekennzeichnet, daß ein Fu`hrungsdraht (30) mit seinem rückwärtigen Ende in ein vorderes Ende des flexiblen Rohres eingesetzt und an diesem so befestigt ist, daß er am Rohr vorsteht.

2. Katheder nach Anspruch 1, dadurch gekennzeichnet, daß der Führungsdraht (30) ein rundes Ende aufweist.

## Revendications

1. Cathéter comprenant un tube souple qui comporte un passage de fluide pour un fluide, ledit tube souple étant un tube fabriqué en une résine de fluoroéthylène, et un revêtement de caoutchouc résistant à la pression pour recouvrir ledit tube souple à l'exception de sa partie d'extrémité, caractérisé en ce qu'un fil de guidage (30) est inséré par son extrémité arrière, dans une extrémité avant dudit tube souple et est fixé à celui-ci de manière à faire saillie par rapport audit tube.

2. Cathéter suivant la revendication 1, dans lequel ledit fil de guidage (30) présente une extrémité arrondie.

## FIG. 1

## FIG. 2

## FIG. 3